# EUROPEAN PATENT APPLICATION

(11) **EP 4 368 132 A1**
(43) Date of publication of application: **15.05.2024**
(21) Application number: 23198534.2
(22) Date of filing: 20.09.2023
(51) Int. Cl.: A61B 18/14, A61B 17/00

(54) **ELECTRODE FOR A HAND-HELD ELECTROSURGICAL INSTRUMENT AND METHOD OF MANUFACTURING AN ELECTRODE**

(30) Priority: 08.11.2022 US 202263423537 P
(71) Applicant: Olympus Winter & Ibe GmbH, 22045 Hamburg (DE)
(72) Inventor: Knopf, Christoph, 23617 Stockelsdorf (DE); Brockmann, Christian, 21279 Hollenstedt (DE); Lavicka, Jiri, 75002 Prerov (CZ); Komínek, Petr, 75002 Prerov (CZ)
(74) Representative: Hoener, Matthias

(57) **Abstract**

The invention provides a method for manufacturing an electrode as well as an electrode which can be used particularly efficiently and can be manufactured in a particularly cost-effective manner. This is achieved in that an electrode (16) for an electrosurgical hand instrument comprises an electrically conductive wire (26) consisting of a plurality of sections. In this case, two sections of the wire R1 and L1, which are directly adjacent to two ends of the wire (26), are aligned parallel to each other as well as straight. Furthermore, the first two sections R1 and L1 are adjoined by two second sections R2 and L2, which are also aligned parallel to each other as well as straight. Thereby, section R2 directly adjoins section R1 and section L2 directly adjoins section L1. The two sections R2 and L2 are in turn connected to each other by a further section C.

## Description

The invention relates to an electrode for an electrosurgical hand-held instrument according to claim 1. Furthermore, the invention relates to a method for manufacturing an electrode according to claim 9.

Electrosurgical hand-held devices, in particular resectoscopes, of the type described are used primarily in urology for electrosurgical work. In this context, these devices are usually used for resection and for evaporation of tissue, for example of tissue in the lower urinary tract. For this purpose, the hand-held device, in particular the resectoscope, may comprise a longitudinally displaceable electrode carrier which, after insertion of the device into the body to be treated, may be advanced with a distal working end from a distal end of the instrument shaft of the hand-held device. An electrosurgical electrode is disposed on the electrode carrier at a distal end thereof. This electrode may be in the form of a loop, for example, and is pulled or pushed through the tissue to manipulate the tissue, depending on the design of the instrument.

For the above application, a high-frequency electric current is applied to the electrode. It is important to avoid the electrode coming into electrical contact with the shaft tube of the handpiece. In the event of such electrical contact, a short circuit could cause a device defect or lead to unpredictable traumatization in the body to be treated. To avoid such short circuits, hand-held devices include an electrically insulating insulating insert; also called an insulating tip, at their distal end regions. In this case, the insulating insert can be attached either to an inner shaft or shaft tube, in which an electrode carrier is guided, or to the outer shaft of the instrument. Since such hand-held instruments can also be designed for multiple use and must therefore be regularly sterilized or autoclaved, the insulating insert is designed to be detachable for cleaning.

In the case of the hand-held instrument described here for minimally invasive treatment of patients, the aim is for the size of the instruments or their cross-section to be as small as possible in order to minimize trauma to the patient during treatment. Similarly, the aim is to perform the procedure in a particularly efficient manner. The choice of electrode is of crucial importance for efficient surgery. Only with the right, application-specific electrode can the optimal treatment goal be achieved. In particular, the effective cross-section of the electrode or the working instrument relative to the cross-section of the instrument can be of decisive importance. However, the effective cross-section or size of the electrode is limited by the shape and diameter of the shaft of the electrosurgical hand-held instrument. Thus, it is impractical for the effective cross-section of the electrode to be larger than the cross-section of the outer circumference of the shaft. However, in known instruments, the space available for the electrode is not optimally utilized. Approaches for better utilization of the available space pursue highly complicated electrode geometries, which on the one hand are very complex and thus expensive to manufacture, and on the other hand require a great deal of effort in quality control.

The invention is therefore based on the problem of creating a process for manufacturing an electrode and an electrode that can be used particularly efficiently and can be manufactured in a particularly cost-effective manner.

A solution to this probelm is described by claim 1. According to this claim, it is provided that an electrode for a hand-held electrosurgical instrument comprises an electrically conductive wire of a plurality of sections. Thereby, two sections of the wire R1 and L1, which are directly adjacent to two ends of the wire, are aligned parallel to each other as well as straight. Furthermore, two second sections R2 and L2, which are also parallel to each other as well as aligned straight, adjoin the first two sections R1 and L1. Thereby, the section R2 directly adjoins the section R1 and the section L2 directly adjoins the section L1. The two sections R2 and L2 are in turn connected to each other by a further section C. The fact that the continuous wire has a mirror-symmetrical structure and always exhibits shape changes in only one spatial dimension makes it particularly easy to manufacture the electrode. The fact that the electrode has no shapes with directional changes from one section to a subsequent section in more than one dimension makes both production and quality control particularly simple. Quality control is simplified in that bends in only one spatial direction can be inspected particularly easily in an automated or partially automated manner, e.g. with camera systems or profile projectors. This simplification of the structure of the electrode according to the invention means that production in particular can be made particularly cost-effective.

Preferably, the length of the sections R2 and L2 is provided to be 0.7 mm to 1.7 mm. The wire thickness of the sections R1 and L1 and/or R2 and L2 and preferably also of further sections can be 0.2 mm to 1.0 mm. It has been shown that this dimensioning is particularly advantageous for efficient treatment of the patient and for particularly cost-effective manufacture of the electrode.

Preferably, the invention further provides that the two second sections R2 and L2 include with the first sections R1 and L1 an angle α of 35° to 120°, preferably 70°, 45° or 110°. Depending on the type of application of the electrode, different angles can be chosen between the two pairs of sections. Sections R1 and R2 and L1 and L2 each lie in a plane, these planes being aligned parallel to one another. By shaping the various sections in this way according to the invention, a large number of different electrode shapes can be produced in a particularly simple and thus inexpensive manner. Furthermore, another embodiment of the invention may provide that bending radii between sections R1 and R2 and L1 and L2 are 0.1 mm to 1 mm.

In particular, it is provided according to the invention that a section C between sections R2 and L2 has a radius of 2.0 mm to 3.6 mm, preferably 2.8 mm. This loop-like shape of the electrode is particularly versatile and also exhibits high stability. Furthermore, it is conceivable that the section C is straight and aligned at a right angle to the sections R2 and L2. Alternatively, it is also conceivable that the section C has a V-shape or is trapezoidal in shape. The section C essentially represents the section of the electrode in which the tissue is manipulated. By a corresponding movement of the electrode carrier, the electrode with the section C is pulled or pushed through the tissue to be manipulated.

Another advantageous embodiment of the invention may provide that the sections R2, L2 and C lie in a plane. Depending on the application of the electrode, it may be advantageous for said sections to have different angles. By using the segmental shape described herein, which is bent into a plurality of sections, various shapes of an electrode can be made in a simple manner using simple means. By varying the aforementioned lengths and angles, a variety of different shapes and sizes can be produced, and without having to adapt the manufacturing process for this purpose.

Preferably, it is also conceivable that the section C is designed as a roller electrode in that a hollow cylinder is plugged onto a straight section C. The hollow cylinder can have a cylindrical shape or a barrel shape. The hollow cylinder can have a cylindrical shape or a barrel shape.

A method for solving said peoblem is described by the measures of claim 7. Accordingly, it is provided that for manufacturing an electrode for an electrosurgical hand-held instrument according to claim 1, several steps are carried out in succession. In a first step, a straight wire is first deformed in such a way that a central section C is formed, for which purpose two free wire ends of the wire are bent towards each other so that the wire ends are positioned parallel to each other. Subsequently, the two wire ends are bent over relative to the section C in the same way as well as in parallel, with the remaining open wire ends forming the sections R1 and L1 (step 2). These deformation steps allow the manufacture of the electrode to be generalized or standardized. By slightly varying the steps mentioned above, a large number of different electrodes can be produced, which can be formed in different ways depending on the field of application. Due to the simple one-dimensional deformation steps of the aforementioned sections, the use of complex tools can be dispensed with. This simplification of production makes the process particularly cost-effective.

In particular, it is envisaged that the two wire ends are bent through an angle of 35° to 120°, preferably 45°, 90° or 110° relative to section C, to sections L1 and R1. By choosing these angles, all applications of the electrode can be realized. In addition, it is possible to optimize the electrode for other, possibly new, applications.

Preferably, in step 2, it may be provided to bend the two wire ends with respect to section C in such a way that two further parallel and straight sections L2 and R2 are formed between section C and the two parallel and straight sections L1 and R1, which lie in the same plane as sections L1 and R1. Accordingly, sections R2 and L2 can be in the same plane as section C and sections L1 and R1 can be in the same plane as sections R2 and L2. The total of five sections are thus located in only two planes, which allows a particularly simple manufacturing process in two steps. This is particularly advantageous both for manufacturing costs and for checking the quality of the electrodes or for quality assurance.

The method according to the invention can provide in step 1 that the section C between the sections R2 and L2 is formed in an application-specific manner. Accordingly, it is conceivable that the section C is round, semicircular, elliptical, polygonal, straight or V-shaped. It is also conceivable that section C is dominated by further components, such as a roller, pin, button or the like.

A preferred embodiment of the invention is described in more detail below with reference to the drawing. In this show:
- Fig. 1: a schematic representation of a hand-held surgical device, in particular a resectoscope,
- Fig. 2: a side view of an electrode,
- Fig. 3: a front view of the electrode according to Fig. 2,
- Fig. 4: a side view of another embodiment of an electrode,
- Fig. 5: a front view of the electrode according to Fig. 4,
- Fig. 6: a side view of another embodiment of an electrode,
- Fig. 7: a front view of the electrode according to Fig. 6,
- Fig. 8a: Depiction of a wire,
- Fig. 8b: Step 1 of a manufacturing process of an electrode, and
- Fig. 8c: Step 2 of the electrode manufacturing process.

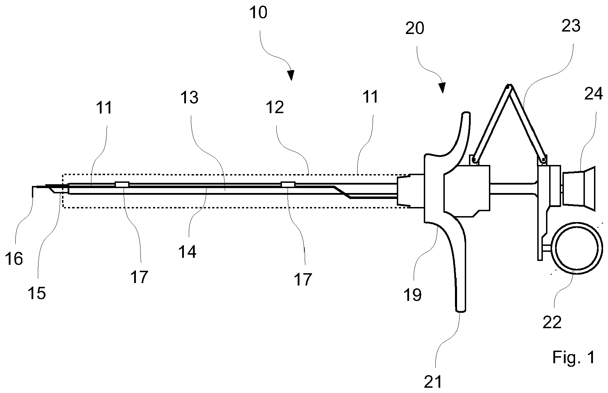

Fig. 1 shows a schematic, side cross-sectional view of a resectoscope known as 10. The resectoscope 10 has a resectoscope shaft 11, which includes an outer shaft 12 or sheath tube shown. A tubular inner shaft 13 extends within the outer shaft 12. A electrode array 14 and an indicated optic 15 are shown within the inner shaft 13. In addition, other elements not shown here may be disposed within the resectoscope 10, such as a separate irrigation tube and the like.

The electrode array 14 has an electrosurgical tool or electrode 16 at a distal end. The electrode 16 shown here is shown as a loop, but may also be formed as a button or the like.

The electrode holder 14 can be forcibly moved axially in the distal and proximal direction by actuating a handle 19. In doing so, it can be pushed beyond the distal end of the inner shaft 13 and the outer shaft 12. This allows the surgeon to manipulate tissue further away from the resectoscope tip. Furthermore, for this purpose, the inner shaft 13 and/or the electrode carrier 14 can be rotatably mounted about their longitudinal axis. For the manipulation of the tissue, a high-frequency electric current is applied to the electrode 16.

The resectoscope 10 shown in Fig. 1 has a passive transporter in which a carriage 20 is displaced in the distal direction against the distal, first handle part 21 by relative movement of the handle parts 21 and 22 arranged proximally on the resectoscope shaft 11 against a spring force applied by a spring bridge 23. When the carriage 20 is displaced in the distal direction against the handle part 21, the electrode support 14 is displaced in the distal direction in a manner not shown. When the load on the handle parts 21, 22 is relieved, the spring force generated by the spring bridge 23 forces the slide 20 back to its initial position, pulling the electrode carrier 14 in the proximal direction. When the slide 20 is moved back, an electrosurgical procedure can be performed with the electrode 16 without any manual force from the operator, i.e. passively.

For targeted treatment by means of the electrode 16, the optics 15 are positioned in such a way that the surgeon has an optimal view of the area of the operation. For this purpose, the resectoscope 10 has an eyepiece 24 at a proximal end, which is connected to the optics 15. Alternatively, it is also conceivable that a camera is arranged on the resectoscope 10 instead of the eyepiece 24.

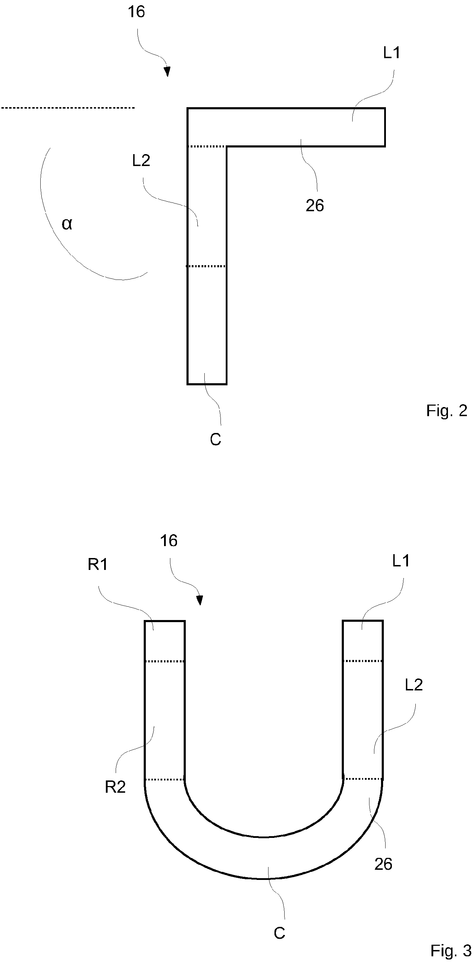

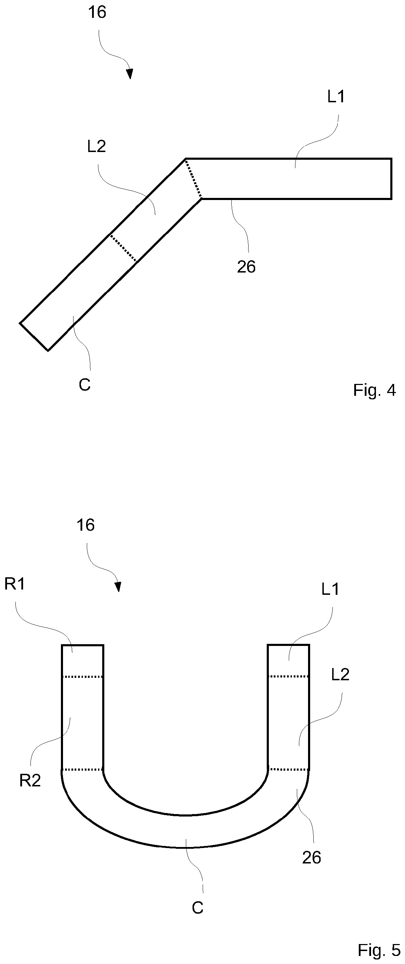

In the following figures, several embodiments of electrodes 16 are shown, all of which follow the same structure. For example, these electrodes each have first sections R1 and L1, with the first sections R1 and L1 being pronounced equal in length and oriented parallel to each other. These first sections R1 and L1 are coupled to the distal ends of the electrode support tubes. This coupling with the electrode support tubes stabilizes the electrode 16 as well as applies electrical energy to it. The first sections R1 and L1 are followed by the two second sections R2 and L2. These two second sections R2 and L2 are also formed with the same length and are aligned parallel to each other. The transition from the first sections R1 and L1 to the second sections L2 and L2 takes place in one plane, i.e. the manufacturing process is particularly simple and no complicated tools are required (see Figs. 2 to 7).

In the embodiment example shown in Figs. 2 to 5, the second sections R2 and L2 are inclined relative to the first sections R1 and L1 by an angle of 90° and 45°, respectively. It is envisaged that this angle α takes a value between 35° and 120°, preferably 45°, 90° or 110°, or any other angle.

Section C is located between the two second sections R2 and L2. This section C connects the two second sections R2 and L2 and is formed as a loop in the embodiment examples shown in Figs. 2 to 5. The shape of the section C can also vary and, for example, have a larger or smaller radius of curvature. In the present embodiment example, the section C lies in the same plane as the sections R2 and L2. To perform the operation, the electrode 16 is pulled or pushed through the tissue to be treated with the section C, depending on the type of resectoscope 10. For this purpose, it is preferably provided that the wire 26 of the electrode 16 has a circular cross-section.

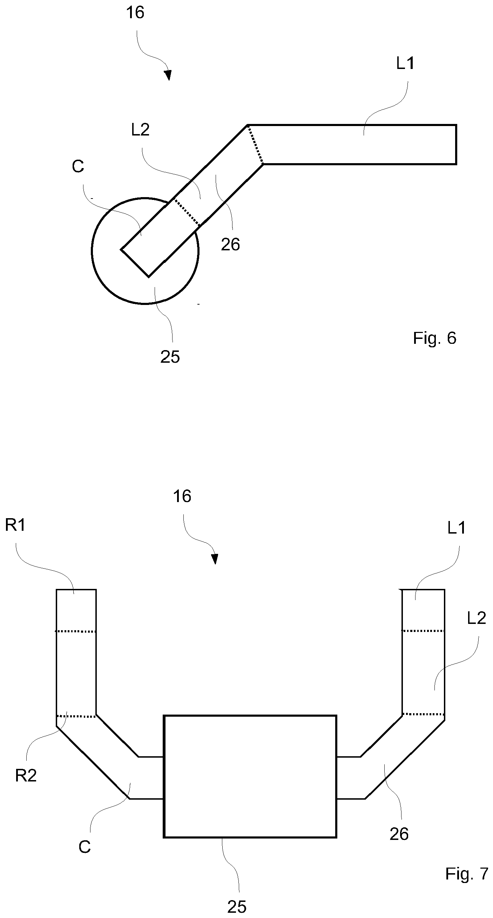

Figs. 6 and 7 show a so-called roller electrode 16. Like the previous electrodes 16, this electrode 16 is also composed of two first sections R1 and L1 and second sections R2 and L2 adjoining the first sections R1 and L1, which are angled with respect to the first sections R1 and L1. Between the two second sections R2 and L2 a trapezoidal section C is arranged, on which a roller 25 is located. This roller 25 is used to treat the tissue to be manipulated during the treatment.

In addition to the embodiment examples shown here, other forms of the electrode 16 are conceivable. However, it is essential that all electrodes 16 are constructed from one wire 26, from which the aforementioned sections are formed, which always have only one change of direction between them in one plane. This prevents the electrode 16 from adopting a complicated geometry, which is particularly difficult to manufacture and particularly difficult to control in terms of quality. The structure described here, consisting of three different sections, means that production can be standardized, resulting in a very cost-effective manufacturing process.

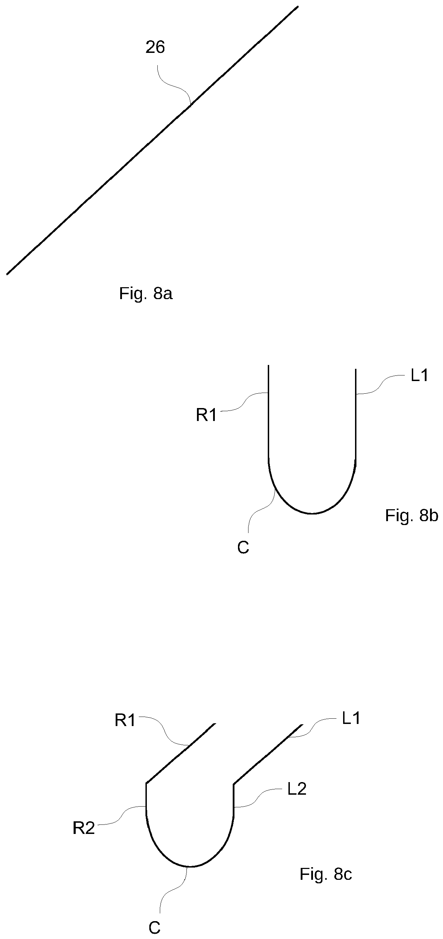

Figs. 8a to 8c show a highly schematized sketch of the manufacturing process for producing the electrode 16 according to the invention. Accordingly, a straight wire 26 (Fig. 8a), which may already have the total length of the electrode 16 or may also be longer, is first bent in the center in a first step, so that the two sections R1 and L1 have the same length and are aligned parallel to each other (Fig. 8b). The section C, which connects the two sections R1 and L1 with each other, can be bent around a shape, which then determines the shape of the section C. The section C can then be bent around the shape of the section L1. This first forming step of the wire 26 is thus performed in a plane. The second forming step of the wire 26 now takes place in a plane which is oriented transversely to the plane determined by the section C. As shown in Fig. 8c, in a second step, sections R1 and L1 are equally bent in a direction transverse to section C, with sections R1 and L1 maintaining their parallel orientation. In the embodiment of the method shown in Fig. 8c, the bending is performed such that straight parallel sections remain between section C and sections R1 and L1. These sections form the second sections R2 and L2. By varying the length of these sections R2 and L2, and also by varying the shape of section C, the shape or effective cross-section of the electrode can be varied. If necessary, after completion of the manufacturing process, the length of sections R1 and L1 can still be adjusted to accommodate the dimension of the electrosurgical hand-held instrument. Similarly, it is conceivable that the free ends of sections R1 and L1 are provided with further contact means to improve the electrical contact to the electrode support tubes.

This means that essentially only two bending steps in two planes are required to manufacture the electrode 16. This not only makes it particularly easy to manufacture the electrode 16, but also to check the quality of the electrode 16.

### List of Reference Numerals:

- 10: Resectoscope
- 11: Resectoscope shaft
- 12: Outer shaft
- 13: Inner shaft
- 14: Electrode carrier
- 15: Optics
- 16: Electrode
- 17: Guide element
- 18: Longitudinal axis
- 19: Handle
- 20: Sled
- 21: Handle part
- 22: Handle part
- 23: Spring bridge
- 24: Ocular
- 25: Roll
- 26: Wire

- R1: first section
- R2: second section
- L1: first section
- L2: second section
- C: central section

- α: Angle

## Claims

1. Electrode (16) for an electrosurgical hand-held instrument, in particular for a resectoscope (10), consisting of an electrically conductive wire (26), wherein the two ends of the wire (26) can be coupled to an electrode support (14) of the hand-held instrument, **characterised in that in that** two sections R1 and L1 of the wire (26), which adjoin the two ends of the wire (26), are aligned parallel to one another and straight, and the first sections R1 and L1 being adjoined by two second sections R2 and L2, which are likewise aligned parallel to one another and straight.

2. Electrode (16) for an electrosurgical hand-held instrument according to claim 1, **characterized in that** the lengths of the sections R2 and L2 are 0.7 mm to 1.7 mm.

3. Electrode (16) for an electrosurgical hand-held instrument according to claim 1 or 2, **characterized in that** the wire thicknesses of the sections R1 and L1 and/or R2 and L2 and preferably also of further sections are 0.2 mm to 1.0 mm.

4. Electrode (16) for an electrosurgical hand-held instrument according to any of the preceding claims, **characterized in that** the two second sections R2 and L2 enclose an angle α of 35° to 120°, preferably 45°, 90° or 110°, with the first sections R1 and L1.

5. Electrode (16) for an electrosurgical hand-held instrument according to any of the preceding claims, **characterized in that** bending radii between sections R1 and R2 and L1 and L2 are 0.1 mm to 1 mm.

6. Electrode (16) for an electrosurgical hand-held instrument according to one of the preceding claims, **characterized in that** a section C extends between the two sections R2 and L2, which section C has a radius of 2.0 mm to 3.6 mm , preferably 2.8 mm, or is straight and is aligned at a right angle to the sections R2 and L2 or has a V-shape or is trapezoidal .

7. Electrode (16) for an electrosurgical hand-held instrument according to claim 6, **characterized in that** the sections R2, L2 and C lie in one plane.

8. Electrode (16) for an electrosurgical hand-held instrument according to claim 6, **characterized in that** the section C forms a roller electrode **in that** a roller (25) is plugged onto a straight section C.

9. Method for producing an electrode (16) for an electrosurgical hand-held instrument, in particular for a resectoscope (10), according to claim 1, wherein the electrode (16) consists of an electrically conductive wire (26) and the two ends of the wire (26) can be coupled to an electrode support (14) of the hand instrument, **characterized in that** first a straight wire (26) is deformed in such a way that a central section C is formed, for which purpose two free wire ends of the wire (26) are bent towards each other so that the wire ends are positioned parallel to each other (step 1), subsequently the two wire ends are bent over relative to the section C equally as well as parallel, the remaining open wire ends forming the sections R1 and L1 (step 2).

10. Method of manufacturing an electrode (16) according to claim 9, **characterized in that** the two wire ends are bent through an angle of 35° to 120°, preferably 45°, 90° or 110° relative to section C, to sections L1 and R1.

11. Method of manufacturing an electrode (16) according to claim 9 or 10, **characterized in that** in step 2 the two wire ends are bent with respect to the section C in such a way that between the section C and the two parallel and straight sections L1 and R1 two further parallel as well as straight sections L2 and R2 are formed which lie in the same plane as the sections L1 and R1.

12. Method of manufacturing an electrode (16) according to any one of claims 9 to 11, **characterized in that** in step 1 the section C is formed in an application-specific round, semicircular, straight, V-shaped or the like manner.
